# EUROPÄISCHE PATENTSCHRIFT

(11) **EP 1 220 654 B1**
(45) Veröffentlichungstag und Bekanntmachung des Hinweises auf die Patenterteilung: **21.12.2005**
(21) Anmeldenummer: 00969400.1
(22) Anmeldetag: 04.10.2000
(51) Int. Cl.: A61K 7/13

(54) **HAARFÄRBEVERFAHREN**
HAIR COLORATION METHOD
PROCEDE DE COLORATION DES CHEVEUX

(30) Priorität: 12.10.1999 DE 19949033
(43) Veröffentlichungstag der Anmeldung: 10.07.2002
(73) Patentinhaber: Henkel Kommanditgesellschaft auf Aktien, 40589 Düsseldorf (DE)
(72) Erfinder: HÖFFKES, Horst, 40595 Düsseldorf (DE); OBERKOBUSCH, Doris, 40591 Düsseldorf (DE); MÖLLER, Hinrich, 40789 Monheim (DE); KLEEN, Astrid, 40699 Erkrath (DE)
(86) Internationale Anmeldenummer: PCT/EP2000/009687
(87) Internationale Veröffentlichungsnummer: WO 2001/026616

(56) Entgegenhaltungen:
- DE-A- 4 409 143
- DE-A- 19 630 274
- DE-A- 19 717 224
- DE-A- 19 842 071
- US-A- 4 425 132
- US-A- 5 431 698

## Beschreibung

Gegenstand der Erfindung ist ein zweistufiges Verfahren zur Färbung von Humanhaar, bei welchem der Haaransatz mit einem üblichen Oxidationshaarfärbemittel unter Verwendung eines Oxidationsmittels und in einem zweiten Schritt die gesamte Haarlänge- oder der verbleibende Bereich des Haars mit einem weniger agressiven Haarfärbemittel, das ein nichtoxidatives Farbmittel gleicher oder ähnlicher Nuance ist, eingefärbt wird.

Die heute üblichen Oxidationshaafärbemittel werden in der Regel bei alkalischen pH-Werten von 8-11 auf das Haar aufgetragen und vor oder nach dem Auftrag auf das Haar mit einem chemischen Oxidationsmittel, meist Wasserstoffperoxid, einer Perverbindung oder einem Wasserstoffperoxid-Addukt, aktiviert, d.h. oxidativ zum Farbstoff polymerisiert. Eine solche Behandlung hat, insbesondere bei wiederholter Anwendung oder wenn das Haar bereits durch andere Behandlungen vorgeschädigt ist, eine Schädigung der Haarstruktur zur Folge, die sich in einer schlechten Kämmbarkeit und Formbarkeit sowie in einem Verlust an Glanz, Elastizität und Reißfestigkeit zeigt. Die Schädigung des Haars nimmt vom Haaransatz, also von dem Bereich, der der Kopfhaut benachbart ist, bis zur Haarspitze - also mit zunehmendem Alter der Keratinsubstanz - kontinuierlich zu, da der jüngere, der Kopfhaut benachbarte Teil des Haars weniger chemischen, mechanischen und atmosphärischen (UV-Licht) Belastungen ausgesetzt war als die älteren Bereiche bis hin zur Haarspitze. Aus diesem Grunde leiden Haarfärbungen oft an ungenügender Egalität, da die porösen, geschädigten Partien des Haars viele Farbstoffe stärker adsorbieren und daher tiefer gefärbt werden als der Bereich des Haaransatzes. In der Praxis wird diesem Übelstand dadurch begegnet, daß man zunächst den Haaransatz einfärbt und nach einer gewissen Einwirkungszeit diesen Farbstoff auch auf der restlichen Haarlänge verteilt. Dadurch wird zwar die Einwirkungszeit der alkalischen Oxidationsmittel auf der Haarlänge verkürzt, es besteht aber immer noch das Problem, daß bei jeder Färbebehandlung erneut ein oxidativer Angriff auf die bereits geschädigte Haarlänge erfolgt

In EP 0624 362 B1 wurde vorgeschlagen, zur Schonung der bereits stärker strapazierten Haarlängen in einem zweistufigen Färbeverfahren den Haaransatz mit einem alkalischen und die gesamte Haarlänge mit einem sauer eingestellten Oxidationshaarfärbemittel zu färben. Diese Arbeitsweise bedeutet aber für den Haaransatz, daß er mehrfach der Einwirkung des Oxidationsmittels ausgesetzt ist und für die bereits vorgeschädigte Haarlänge, daß sie bei jedem Färbevorgang erneut mit dem Oxidationsmittel in Kontakt kommt.

Die Erfinder haben sich daher die Aufgabe gestellt, ein Verfahren zu entwickeln, daß die Schädigung der strapazierten Haarlängen beim Haarefärben weiter verringert. Als Lösung dieser Aufgabe wird ein Verfahren zur schonenden Färbung der Haare in zwei Farbestufen vorgeschlagen, bei dem man das Haar in der ersten Stufe im Bereich des Haaransatzes mit einem Oxidationshaarfärbemittel unter Verwendung eines chemischen Oxidationsmittels und in einer zweiten Stufe in der gesamten Länge, oder wenigstens in dem verbleibenden Längenbereich, mit einem nachfolgend beschriebenen nichtoxidativen Haarfärbesystem gleicher oder ähnlicher Nuance einfärbt.

Ein solches Verfahren hat den Vorteil, daß die stärker vorgeschädigten Haarlängen nicht immer wieder mit einer Zusammensetzung behandelt werden, die ein chemisches Oxidationsmittel enthalten. Das Verfahren eignet sich ganz besonders für Personen, die sich in regelmäßigen Zeitabständen das Haar in der gleichen oder ähnlichen Nuance nachfärben, insbesondere um dem frisch nachgewachsenen Haaransatz auf den gewünschten Farbton zu bringen und bei den Haarlängen lediglich die durch Haarewaschen und Witterung bedingten Farbstoffverluste auszugleichen.

Gegenstand ist ein Verfahren zur schonenden Färbung der Haare in zwei Stufen, dadurch gekennzeichnet, daß man das Haar in der ersten Stufe im Bereich des Haaransatzes mit einem Oxidationshaarfärbemittel unter Verwendung eines chemischen Oxidationsmittels und in einer zweiten Stufe in der gesamten Haarlänge oder wenigstens in dem verbleibenden Längenbereich mit einem nichtoxidativen Haarfärbemittel, enthaltend reaktive Carbonylverbindungen in Kombination mit Amino- oder Hydroxylverbindungen, gleicher oder ähnlicher Nuance einfärbt.

Das für die erste Färbestufe zu verwendende Oxidationshaarfärbemittel kann prinzipiell jedes der im Stande der Technik bekannten und in der Praxis üblichen Oxidationsfärbemittel sein. Solche Zubereitungen enthalten

Oxidationsfarbstoffvorprodukte in einem wäßrigen Träger und werden unmittelbar vor der Anwendung mit einer Oxidationsmittelzubereitung vermischt. In dem dabei gebildeten Färbeansatz wird durch das Oxidationsmittel eine Oxidation der sogenannten Entwicklerverbindungen zu polymerisierbaren Zwischenstufen eingeleitet, die durch Polymerisation - gegebenenfalls unter Einbau der sogenannten Kupplerverbindungen (color modifier) - zum eigentlichen Farbstoff abreagieren.

In der Regel gelingt es nicht, allein mit einer Entwicklerkomponente eine auf dem Haar natürlich wirkende Farbnuance zu erhalten. In der Praxis werden daher meist Kombinationen mit Kupplerkomponenten und mit anderen Entwicklerkomponenten eingesetzt.

Die als Oxidationsfärbemittelvorprodukte bekannten Entwicklerverbindungen sind üblicherweise primäre aromatische Amine, die wenigstens eine weitere, in para- oder ortho-Position befindliche freie oder substituierte Amino-oder Hydroxylgruppe aufweisen. Weitere bekannte Entwickler sind heterocyclische Hydrazone, 4-Aminopyrazolon-Derivate, 2,4,5,6-Tetraaminopyrimidin und dessen Derivate und Analoga.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, 4,4'-Diaminodiphenylamin, 4-Amino-3-fluorphenol, 2-Amino-methyl-4-aminophenol, 2-Diethylaminomethyl-4-aminophenol, Bis-(2-hydroxy-5-amino-phenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z. B. 4,5-Diamino-1-(2'-hydroxyethyl)-pyrazol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin sowie 4-Hydroxy-2,5,6-triaminopyrimidin.

Zur Nuancierung der erzielbaren Farbtöne können die erfindungsgemäßen Mittel weiterhin noch eine oder mehrere Kupplerkomponenten enthalten. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 2-Hydroxy-4-aminophenoxyethanol, 2-Methyl-5-(2-hydroxyethylamino)-phenol und 2,6-Dihydroxy-3,4-dimethylpyridin.

Besonders bevorzugte Kuppler-Komponenten sind 1-Naphthol, 1,5-, 2,7- und 1,7-Dihydroxynaphthalin, 5-Amino-2-methylphenol, Resorcin, 3-Aminophenol, 4-Chlorresorcin, 2-Chlor-6-methyl-3-aminophenol, 2-Methylresorcin, 5-Methylresorcin, 2,5-Dimethylresorcin sowie 2,6-Dihydroxy-3,4-diaminopyridin.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Üblicherweise werden Entwicklerkomponenten und Kupplerkomponenten in etwa gleichen molaren Mengen zueinander eingesetzt. Wenn sich auch der äquimolare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

Gemäß einer weiteren bevorzugten Ausführungsform des Gegenstandes der vorliegenden Erfindung kann das Farbstoffvorprodukt ein Derivat des Indolins der Formel (Ia) sein, 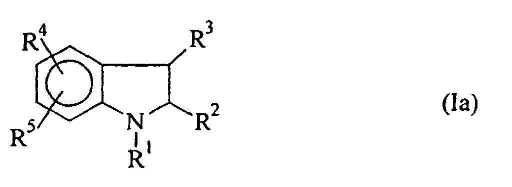 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, daß R⁴ und R⁵ nicht gleichzeitig Wasserstoff sind.

In einer dritten bevorzugten Ausführungsform dieses Gegenstandes der vorliegenden Erfindung kann das Farbstoffvorprodukt ein Derivat des Indols der Formel (Ib) sein, 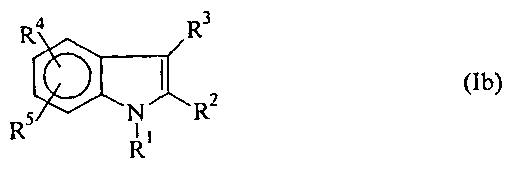 in der unabhängig voneinander R¹ steht für Wasserstoff, eine C₁- bis C₄-Alkylgruppe oder eine C₁- bis C₄-Hydroxy-alkylgruppe, R² steht für Wasserstoff oder eine -COOH-Gruppe, wobei die -COOH-Gruppe auch als Salz mit einem physiologisch verträglichen Kation vorliegen kann, R³ steht für Wasserstoff oder eine C₁- bis C₄-Alkylgruppe, R⁴ steht für Wasserstoff, eine Hydroxygruppe, eine Aminogruppe, eine C₁- bis C₄-Alkoxygruppe oder eine Gruppe -OCO-R⁶, in der R⁶ steht für eine C₁- bis C₄-Alkylgruppe, und R⁵ steht für eine der unter R⁴ genannten Gruppen, oder ein physiologisch verträgliches Salz dieser Verbindungen mit einer organischen oder anorganischen Säure, mit der Maßgabe, daß R⁴ und R⁵ nicht gleichzeitig Wasserstoff sind.

Bevorzugte Stoffe der Formel (Ia) sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin. Bevorzugte Stoffe der Formel (Ib) sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Ganz besonders bevorzugt sind 5,6-Dihydroxyindol sowie 5,6-Dihydroxyindolin in Form ihrer mineralsauren Salze.

In einer ersten bevorzugten Variante der oben beschriebenen Ausführungsformen werden die Mittel derart formuliert, daß sie als Farbstoffvorprodukte nur Indol- und/oder Indolinderivate der Formeln (Ia) und (Ib) enthalten und frei sind von üblichen Oxidationsfarbstoffvorprodukten vom Entwickler- bzw. Kupplertyp.

In einer zweiten bevorzugten Variante der oben beschriebenen Ausführungsformen können die erfindungsgemäßen Mittel neben den Indol- und/oder Indolinderivaten der Formeln (Ia) und (Ib) auch noch übliche Oxidationsfarbstoffvorprodukte vom Entwickler- bzw. Kupplertyp enthalten.

Es kann erfindungsgemäß besonders bevorzugt sein, die Indol- und/oder die Indolinderivate der Formeln (Ia) und (Ib) in Kombination mit einer oder mehrere Kupplerkomponenten in Haarfärbemitteln einzusetzen. Beispielhaft sei an dieser Stelle ausdrücklich auf die oben genannten Kupplerkomponenten verwiesen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248 - 250; direktziehende Farbstoffe) sowie Kapitel 8, Seiten 264 - 267 (Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsuntemehmen für Arzneimittel, Reformwaren und Körperpflegemittel e.V., Mannheim, Bezug genommen.

Die meisten Oxidationsfärbemittel benötigen zur Einleitung der Oxidation und zur Ausbildung des Farbstoffs in einem für die Haarfärbung tragbaren Zeitraum ein chemisches Oxidationsmittel. Unter diesem Begriff soll im Rahmen dieser Erfindung ein Stoff verstanden werden, der (auch in Abwesenheit von Luftsauerstoff) die oxidative Entwicklung der Oxidationsfärbung einleiten kann. Beispiele für solche Stoffe sind Wasserstoffperoxid und dessen Anlagerungsprodukte an z. B. Harnstoff, Polyvinylpyrrolidon oder Melanin, Salze der Peroxydischwefelsäure, Perborate, Percarbonate, Perjodate, Permanganate, Hypochlorite, Chlorite, Eisen (III)-salze, Bichromat, Silberoxid, organische Percarbonsäuren und organische Peroxide.

Die Oxidationsfarbstoff-Vorprodukte werden in einen geeigneten Träger zur Aufbringung auf das Haar eingearbeitet. Solche, für das erfindungsgemäße Verfahren geeignete Träger sind bevorzugt Emulsionen und Gele.

Auch die chemischen Oxidationsmittel werden in geeignete Träger, für wasserlösliche Produkte bevorzugt Emulsionen oder Gele, eingearbeitet. Pulverförmige Produkte können auch zusammen mit inerten Trägerpulvern, z. B. mit Kieselsäure, Stärkepulver oder Holzmehl, formuliert werden.

Vor der Anwendung werden die Zubereitung der Oxidationsfarbstoff-Vorprodukte und die Zubereitung der Oxidationsmittel miteinander gemischt und der dabei gebildete Färbeansatz auf den Haaransatz aufgetragen. Hierzu eignet sich bevorzugt eine Applikationsbürste oder ein saugfähiges Substrat (Filz oder Schwamm), das für den Auftrag eines wäßrigen Präparats auf Teilbereiche des Haars geeignet ist.

Nach einer Einwirkungszeit, bevorzugt von 3-30 Minuten, wird das Haar mit Wasser oder mit einem üblichen Haarwaschmittel gewaschen und gespült.

Für die zweite Stufe des erfindungsgemäßen Haarfärbeverfahrens stehen nachfolgende nichtoxidative Färbesysteme zur Verfügung, die für die Entwicklung einer Färbung kein chemisches Oxidationsmittel benötigen.

Die Gruppe von nichtoxidativen Farbstoffen für die Ausführung der zweiten Stufe des erfindungsgemäßen Verfahrens sind die Haarfarbstoffe vom Typ der reaktiven Carbonylverbindungen. Solche Farbstoffe sind seit längerem bekannt:

Geeignete Verbindungen vom Typ der aromatischen Aldehyde sind z. B. in der deutschen Patentanmeldung DE 196 30 274 A1 und DE 196 30 275 A1 beschrieben. Geeignete Verbindungen sind z. B. der 2-Hydroxybenzaldehyd, der 4-Hydroxy-3-methoxybenzaldehyd (Vanillin) und der 4-Hydroxy-3-methoxy-cinnamaldehyd (Coniferylaldehyd). Weitere geeignete Arylaldehyde sind aus US 5,199,954 bekannt.

Geeignete Verbindungen vom Typ der heteroaromatischen Aldehyde sind z. B. in der deutschen Patentanmeldung DE 197 172 80.6 beschrieben. Besonders gut geeignete Farbstoffe für das erfindungsgemäße Verfahren sind z. B. trans-β-(2-Furyl)-acrolein, 1-Methylindol-3-aldehyd, 2-(1,3,3-Trimethyl-2-indolinyliden)-acetaldehyd oder Antipyrin-4-aldehyd. Spezielle Produkte dieses Typs mit einer Pyridinium-Gruppe sind in der deutschen Patentanmeldung DE 197 45 356.2 beschrieben, z. B. die sehr gut geeigneten 4-Formyl-1-methylpyridinium-benzolsulfonat und 4-Formyl-1-methylchinolinium-methan-sulfonat bzw. -methylsulfat bzw. -p-toluolsulfonat sowie das 2-Formyl-1-methylchinolinium-p-toluolsulfonat-hydrat.

Geeignete Farbstoffe vom Typ der ungesättigten Aldehyde sind z. B. in der deutschen Patentanmeldung DE 197 17 224.5 beschrieben. Für die vorliegende Erfindung eignet sich besonders gut der Glutaconaldehyd in Form seiner Salze, z. B. seines Alkali- oder Tetrabutylammoniumsalzes oder der 2-Chlor-3-hydroxymethylen-1-cyclohexen-1-aldehyd.

Dialdehyde und Diketone und deren Derivate, die sich als Farbstoffe für das erfindungsgemäße Verfahren eignen, sind z. B. cyclische 1,2- und 1,3-Dicarbonyl-verbindungen, wie Isatin, Ninhydrin, Alloxan, Isobarbitursäure, p- und o-Chinone, 1,3-Indandione und deren Derivate. Solche Farbstoffe finden sich z. B. in der deutschen Offenlegungsschrift DE 43 35 627 A1. Geeignete Verbindungen sind z. B. der Malon-dialdehyd, bevorzugt in Form seines Dimethylacetals, das 2-Nitro-1,3-indandion oder das 2-Acetyl-1,3-cyclohexandion. Eine geeignete 1,2-Dicarbonylverbindung ist auch das Isatinsäure-Kaliumsalz.

Zu den erfindungsgemäß geeigneten Diketonen gehören auch cyclische Dicarbonylverbindungen wie z. B. das Isatin und dessen Derivate, wie sie z. B. in der deutschen Offenlegungsschrift DE 44 09 143 A 1 beschrieben sind. Für das erfindungsgemäße Verfahren sind z. B. das Isatin-5-Sulfonsäure-Kaliumsalz, das N-Allylisatin, das 1-Piperidinomethylisatin, das 1-Hydroxymethylisatin und das 1-Diethylaminomethylisatin geeignet.

Eine weitere geeignete cyclische Dicarbonylverbindung ist z. B. auch die Dehydroascorbinsäure, deren Eignung als Haarfarbstoff aus der deutschen Patentanmeldung DE 197 45 354.6 bekannt ist. Schließlich eignen sich auch die Acetale, Iminderivate und Halbaminale der genannten reaktiven Carbonylverbindungen. Solche Verbindungen werden durch Reaktion der Carboxylgruppe mit primären Alkoholen oder Aminen und ggf. Wasserabspaltung erhalten.

Ausgehend von den ungesättigten Dialdehyden und Diketonen gelangt man dabei in die Gruppe der Merocyanin- und Cyanin- bzw. Azomethin-Farbstoffe. Geeignete IminDerivate des Glutacondialdehyds sind z. B. das Mono-N-methylanilin-Derivat des Glutaconaldehyds (5-N-Methylanilinopentadienal) oder das N-(5-Anilino-2,4-pentadien-1-yliden)-anilinium-chlorid. Ein weiterer geeigneter vinyloger Cyaninfarbstoff ist das 7-Dimethylamino-2,4,6-heptatrienyliden-dimethylammonium-perchlorat. Solche Verbindungen sind als Haarfärbe-mittel-Komponenten z. B. aus der deutschen Patentanmeldung DE 197 17 223.7 bekannt.

Viele der genannten reaktiven Carbonylverbindungen färben keratinhaltige Fasern unter Ausprägung verschiedenster Farbnuancen erst in Kombination mit einer oder mehreren Verbindungen mit primärer oder sekundärer Amino- oder Hydroxygruppe, ausgewählt aus der Gruppe der Aminosäuren und Peptide, der aromatischen Amine, Phenole, Aminophenole, stickstoffhaltigen Heterocyclen sowie CH-aktiven Verbindungen.

Dabei werden in vielen Fällen auch dunklere Nuancen erhalten.

Geeignete Aminosäuren sind z. B. die natürlich vorkommenden und synthetischen Aminosäuren, z. B. Arginin, Histidin, Phenylalanin, Dihydroxyphenylalanin, Ornithin, Lysin. Geeignete Peptide sind vor allem Oligo- und Polypeptide, die eine ausreichende Wasserlöslichkeit in den erfindungsgemäßen Zubereitungen zur Keratinreduktion aufweisen. Als Beispiele sind z. B. Glutathion oder die in den Hydrolysaten von Kollagen, Keratin, Elastin, Casein, Pflanzenproteinen wie Sojaprotein, Weizengluten, Algenprotein oder Mandelprotein enthaltenen Oligopeptide zu nennen.

Geeignete aromatische Amine und Aminophenole sind N,N-Dimethyl-, N,N-Diethyl-, N-(2-Hydroxyethyl)-N-ethyl-, N,N-Bis-(2-hydroxyethyl)-, N-(2-Methoxyethyl-), 2-Chlor-, 2,3-, 2,4- und 2,5-Dichlor-p-phenylendiamin, 2,5-Dihydroxy-4-morpholinoanilin-dihydro-bromid, 2-, 3- und 4-Aminophenol, 2-Aminomethyl-4-aminophenol, 2-Hydroxymethyl-4-aminophenol, o- und p-Phenylendiamin, o- und m-Toluylendiamin, 2,5-Diamino-phenol, -toluol und -phenethol, 4-Amino-3-methylphenol, 2-(2,5-Diaminophenyl)-ethanol, 2,4-Diaminophenoxy-ethanol, 2-(2,5-Diaminophenoxy)-ethanol, 4-Methylamino-, 3-Amino-4-(2'hydroxyethyloxy)-, 3,4-Methylendiamino- und 3,4-Methylendioxyanilin, 3-Amino-2,4-dichlor-. 4-Methylamino-, 2-Methyl-5-amino-, 3-Methyl-4-amino-, 2-Methyl-5-(2-hydro-xyethylamino)-, 6-Methyl-3-amino-2-chlor-, 2-Methyl-5-amino-4-chlor-, 3,4-Methylen-dioxy-, 5-(2-Hydroxyethylamino)-4-methoxy-2-methyl- und 4-Amino-2-hydroxymethyl-phenol, 1,3-Diamino-2,4-dimethoxybenzol, 2-,3-, 4-Aminobenzoesäure, -phenylessig-säure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest, wie sie in der Formel I dargestellt sind 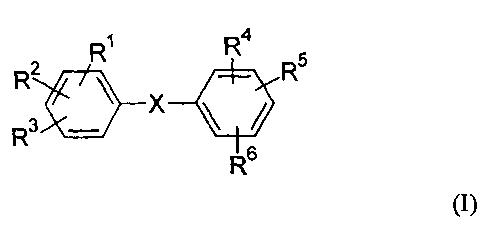 in der R¹ für eine Hydroxy- oder eine Aminogruppe, die durch C₁₋₄-Alkyl, C₁₋₄-Hydroxyalkyl- oder C₁₋₄-Alkoxy-C₁₋₄-alkyl substituiert sein kann, steht,
R², R³, R⁴, R⁵ und R⁶ für Wasserstoff, eine Hydroxy- oder eine Aminogruppe, die durch C₁₋₄-Alky)-, C₁₋₄-Hydroxyalkyl, C₁₋₄-Aminoalkyl- oder C₁₋₄-Alkoxy-C₁₋₄-alkylgruppe substituiert sein kann, für eine Carbon- oder Sulfonsäuregruppe stehen, und
X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel II

Z-(CH₂-Y-CH₂-Z')ₒ (II),

in der Y eine direkte Bindung, eine CH₂- oder CHOH-Gruppe bedeutet,
Z und Z' unabhängig voneinander ein Sauerstoffatom, eine NR⁷-Gruppe, worin R⁷ Wasserstoff, eine C₁₋₄-Alkyl- oder Hydroxy-C₁₋₄-alkylgruppe bedeutet, die Gruppe -O-(CH₂)ₚ-NH oder NH-(CH₂)_{p'}-O, worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-mono- oder -di-Na-Salz, 4,4'-Diaminodiphenylmethan, -sulfid, -sulfoxid, -amin, 4,4'-Diaminodiphenyl-amin-2-sulfonsäure, 4,4'-Diaminobenzophenon, -diphenylether, 3,3',4,4'-Tetraaminodi-phenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propa-nol, 1,3-Bis-[N-(4-aminophenyl)-2-hydroxyethylamino]-2-propanol, N,N-Bis-[2-(4-amino-phenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete Phenole sind z. B. das 2-, 3- oder 4-Methoxy-, das 3-Dimethylamino-, 2-(2-Hydroxyethyl)- und das 3,4-Methylendioxy-phenol, das Resorcin und das 2-, 4- und 5-Methylresorcin, das 2- und 4-Chlorresorcin, 2,5-Dimethylresorcin, Brenzkatechin. Hydrochinon, Pyrogallol, Phloroglucin, Hydroxyhydrochinon, die 2,4- oder 3,4-Dihydroxybenzoe- oder -phenylessigsäure, die Gallussäure, die 2,4,6-Trihydroxybenzoesäure oder das 2,4,5-Trihydroxyacetophenon, das 1-Naphthol, das 1,5-, 2,3- und 2,7-Dihydroxynaphthalin, die 6-Dimethylamino-4-hydroxy-2-naphthalinsulfonsäure oder die 3,6-Dihydroxy-2,7-naphthalindisulfonsäure.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino- und 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino- und 2-Amino-4-methoxy-6-methyl-pyrimidin, 3-Amino-, 3-Amino-5-hydroxy- und 3,5-Diaminopyrazol, 2-,3-, 8-Aminochinolin, 4-Amino-chinaldin, 2-, 6-Aminonicotinsäure, 5-Aminoisochinolin, 5-, 6-Aminoindazol, 5- und 7-Amino-benzimidazol und -benzothiazol, 2,5-Dihydroxy-4-morpholinoanilin sowie Indol- und Indolinderivate, wie 4-, 5-, 6- und 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Diese Färbesysteme können noch weiter verstärkt werden durch geeignete stickstoffhaltige Heterocyclen wie z. B. Piperidin, Piperidin-2-, -3- oder -4-carbonsäure, Pyridin, 2-, 3- oder 4-Hydroxypyridin, Imidazol, 1-Methylimidazol, Histidin, Pyrrolidin, Pyrrolidon, Pyrrolidon-5-carbonsäure, Pyrazol, 1,2,4-Triazol, Piperazin sowie deren physiologisch verträgliche Salze.

Auch CH-aktive Verbindungen können zur Farbentwicklung mit reaktiven Carbonylverbin-dungen verwendet werden. Geeignete CH-aktive Verbindungen sind z. B. 1,2,3,3-Tetramethyl-3H-indoliumiodid, 1,2,3,3-tetramethyl-3H-indolium-p-toluolsylfonat, 1,2,3,3-Tetramethyl-3H-indolium-methansulfonat, Fischersche Base (1,3,3-Trimethyl-2-methylen-indolin), 2,3-Dimethyl-benzothiazoliumiodid, 2,3-Dimethyl-benzothiazoliump-toluolsulfonat, Rhodamin, Rhodanin-3-essigsäure, 1-Ethyl-2-chinaldinium-iodid, 1,4-Dimethylchinolium-iodid, 1-Methyl-2-chinaldinium-iodid, 1,4-Dimethylchinoliniumiodid, Barbitursäure, Thiobarbitursäure, 1,3-Dimethylthiobarbitursäure, 1,3-Diethylthiobarbitursäure, Diethylthio-barbitursäre, Oxindol, 3-Indoxylacetat, Cumaranon und 1-Methyl-3-phenyl-2-parazolinon.

In der Färbemittelzubereitung für die zweite Stufe des erfindungsgemäßen Verfahrens können mehrere Farbstoffe vom Typ der reaktiven Carbonylverbindungen gleichzeitig enthalten sein. Desgleichen können auch mehrere Verbindungen aus der Gruppe der Aminosäuren, Peptide, aromatischen Amine, Aminophenole, Phenole und stickstoffhaltigen Heterocyclen gemeinsam enthalten sein, wenn dies zur Erzielung der gewünschten Farbnuance erforderlich ist.

Das in der zweiten Stufe zur Längenfärbung verwendete Haarfärbemittel wird ausgewählt aus einem Färbesystem aus reaktiven Carbonylverbindungen in Kombination mit farbverstärkenden Amino- oder Hydroxylverbindungen.

Wahrend die in der ersten Stufe zur Ansatzfärbung verwendeten Oxidationshaarfärbemittel ihre intensivste Farbeleistung in einen alkalischen pH-Bereich von 8-11 erreichen können die in der zweiten Stufe zur Längenfärbung verwendeten Haafärbezubereitungen einen schwach sauren bis neutralen pH-Wert im Bereich von 4-7 aufweisen. Diese Angaben beziehen sich jeweils auf den gebrauchsfertigen Färbeansatz, also für die erste Stufe nach dem Zusatz der Oxidationsmittelzubereitung und für die zweite Färbestufe nach dem Zusatz der Farbverstärker oder Katalysator-Zubereitungen.

Das erfindungsgemäße Verfahren zur schonenden Färbung der Haare in zwei Stufen benötigt zu seiner Durchführung verschiedene Zubereitungen, die, insbesondere für die Heimanwendung, bevorzugt getrennt verpackt und zu einer Verkaufseinheit zusammengefaßt sind. Dies hat den Vorteil, daß die Komponenten in den für die Anwendung optimalen Mengenverhältnissen vorbereitet und mit einer einfachen Gebrauchsanleitung ausgestattet werden können und bis zur unmittelbaren Vereinigung zum Färbeansatz getrennt bleiben können, so daß eine vorzeitige Einleitung der Farbstoffentwicklung vermieden wird.

Ein weiterer Gegenstand der Erfindung ist daher ein Haarfärbemittel zur Durchführung des erfindungsgemäßen Verfahrens bestehend aus den getrennt verpackten, zu einer verkaufseinheit zusammengefaßten Komponenten.
(A) einer Zubereitung von Oxidationsfarbstoffvorprodukten
(B) einer Oxidationsmittel-Zubereitung
(C¹) einer Zubereitung von reaktiven Carbonylverbindungen und
(D¹) einer Zubereitung von farbverstärkenden Amino- oder Hydroxylverbindungen

Die Anwendung der erfindungsgemäßen Haarfärbemittel erfolgt bevorzugt in der Weise, daß man zunächst die Farbstoffzubereitung (A) mit der Oxidationsmittelzubereitung (B) vermischt und mit Hilfe eines Applikationswerkzeuges, bevorzugt einer Bürste, auf den Haaransatz, bzw. auf den der Kopfhaut benachbarten Bereich des Haars, etwa in einer Länge von 1-3 cm Länge aufträgt und 10-40 Minuten einwirken läßt. Nach dieser Zeit wird überschüssiger Farbstoff vom Haar, gegebenenfalls unter Verwendung eines Shampoos, abgespült.

Dann wird die nichtoxidative Farbstoffzubereitung C¹, gegebenenfalls nach Vermischen mit dem Farbverstärker D¹ auf die gesamte Länge oder auf den nichtgefärbten Teil des Haares aufgetragen.

Für den Auftrag des Färbeansatzes aus den Komponenten (A) und (B) mit einer Applikationsbürste auf den Haaransatz ist es vorteilhaft, wenn dieser Färbeansatz eine gewisse Viskosität aufweist, die ein Herablaufen des Färbemittels vom Haar oder eine Anfärbung der Kopfhaut verhindert. Aus diesem Grund eignet sich als Träger für die Oxidationsfarbstoffvorprodukte (A) bevorzugt ein Gel oder eine Creme.

ErfindungsgemäB geeignete Gele enthalten bevorzugt Alkali- oder Ammoniumseifen von C₁₂-C₂₂-Fettsäuren als Gelbildner.

Erfindungsgemäß geeignete Färbecremes enthalten zur Verdickung bevorzugt emulgierte Fettkomponenten, z. B. Fettalkohole mit 12-18 C-Atomen, Fettsäurepartialgyceride oder emulgierte kosmetische Ölkomponenten wie z. B. Paraffinöle, Pflanzenöle oder synthetische Esteröle.

Darüber hinaus können solche Zubereitungen alle in der Oxidations-HaarFärberei üblichen Hilfs- und Zusatzstoffe enthalten, dazu gehören vor allem
- Tenside, insbesondere Emulgatoren
- Reduktionsmittel wie z. B. Ascorbinsäure Thioglycolsäure oder Natriumsulfit,
- Puffersubstanzen wie z. B. (NH₄)H₂PO₄, NH₄Cl, NH₃, Alkanolamine
- organische Lösungsmittel wie z. B. Ethanol, 1,2-Propylenglycol oder Glycerin, Diethylenglycol,
- haarkosmetische Wirksubstanzen, wie z. B. Antischuppenwirkstoffe, Vitamine, Pflanzenextrakte, Ceramide, Allantoin, Panthenol, Bisabolol, Pyrrolidoncarboxylat, Lichtschutzmittel (UV-Filter), Proteine und Proteinderivate, Aminosäuren, Cholesterin, Strukturanten wie z. B. Zucker,
- wasserlösliche Verdickungsmittel wie z. B. Pflanzengummi, Xanthan-Gum, Cellulose-Derivate, Stärkeether, Polyvinylalkohol, Polyvinylpyrrolidon, Polyethylenglycole
- Komplexbildner wie z. B. EDTA, NTA oder Acetophosphonsäuren
- Duftstoffe

Die Oxidationsmittel-Zubereitung (B) kann entweder eine wäßrige Lösung, eine Öl-in-Wasser-Emulsion oder eine pulverförmige Zubereitung, z. B. von Harnstoff- oder Melamin-Perhydrat, sein.
Es ist daher darauf zu achten, daß die Mischung der Oxidationsmittelzubereitung (B) mit der Farbstoffzubereitung (A) problemlos und mit wenig Rühraufwand möglich ist und die Viskosität des Färbeansatzes ausreichend hoch für die Ansatzfärbung bleibt.

Die Zubereitungen zur Durchführung der zweiten Stufe des erfindungsgemäßen Haarfärbeverfahrens können als Träger für die nichtoxidativen Farbstoffe (C¹) und die farbverstärkenden Amino- oder Hydroxylverbindungen (D¹) ebenfalls Gele oder Cremes der beschriebenen Art enthalten.

Diese Zubereitungen können aber nach Vereinigung von C¹ und D¹ zum Färbeansatz auch in dünnflüssiger Form, z. B. als Schaumlotion oder Färbeshampoo vorliegen, da eine gleichmäßige Durchfärbung der gesamten Haarlänge auch mit solchen Zubereitungen möglich ist.

Die folgenden Beispiele sollen den Gegenstand der Erfindung näher erläutern:

### Beispiele

| 1 (A) Färbecremes für die Ansatzcoloration | | |
|---|---|---|
| **Nuancen:** | **Kupfergold** | **Mahagoni** |
| Texapon® NSO | 12,0 Gew.% | 12,0 Gew.% |
| Dehyton® K | 10,5 | 10,5 |
| Kokosfettalkohol C₁₂₋₁₈ | 2,0 | 2,0 |
| Cetyl-/Stearylalkohol | 8,7 | 8,5 |
| Eumulgin® B2 | 0,75 | 0,75 |
| Ascorbinsäure | 0,4 | 0,4 |
| Na-Sulfit | 0,4 | 0,4 |
| (NH₄)H₂ PO₄ | 0,8 | 0,8 |
| 2,4,5,6-Tetraaminoyrimidinsulfat | ― | 0,52 |
| p-Toluylendiamin-sulfat | 0,05 | 0,52 |
| 2,7-Dihydroxynaphtalin | - | 0,25 |
| 2-Methylresorcin | 0,02 | - |
| 3-Methyl-4-aminophenol | 0,03 | 0,9 |
| 4-Chlorresorcin | - | 0,15 |
| Resorcin | - | - |
| 1,3-Bis-(2,4-diaminophenoxy)-propan | - | - |
| 2-Amino-3-hydroxypyridin | 0,01 | - |
| 2-Amino-6-chlor-4-nitrophenol | - | 0,1 |
| Wasser, Ammoniak bis pH=10 | ad 100 | ad 100 |

| 1 (B) Oxidationsmittelzubereitung | |
|---|---|
| Wasserstoffperoxid (50 %-ig) | 12,0 Gew. % |
| Texapon® NSO | 2,0 Gew. % |
| Latekoll®B | 16,0 Gew. % |
| 1-Hydroxyethan-1,1-diphosphonsäure | 0,3 Gew.% |
| Na₂H₂P₄O₇ | 0,003 Gew. % |
| Wasser, Ammoniak bis pH = 4 | ad 100 |

### Färbecremes für die Längencoloration

| 1 (C) Direktfarbstoff-Zubereitung | | |
|---|---|---|
| | Kupfergold | Mahagoni |
| 2,4-Dinitrobenzaldehyd | 3,92 Gew.% | - |
| 5-Isatinsulfonsäure, Na-Salz | - | 4,96 Gew. % |
| Variaminblausalz RT | - | - |
| Natrosol® 250 HR | 2,0 Gew.% | 2,0 Gew.% |
| Wasser | ad 100 Gew. % | ad 100 Gew. % |

| 1 (D) Farbverstärker-Zubereitung | | |
|---|---|---|
| | Kupfergold | Mahagoni |
| Texapon® NSO | 52,0 | 52,0 |
| Kokosfettalkohol C₁₂-C₁₈ | 4,0 | 4,0 |
| Cetyl-/Stearylalkohol | 17,0 | 17,0 |
| Ascorbinsäure | 0,1 | 0,1 |
| Na-Sulfit | 0,1 | 0,1 |
| 3,5-Diamino-2,6-dimehoxypyridin 2HCl | 4,84 | - |
| p-Toluylendiamin-sulfat | - | 2,0 |
| 3-Methyl-p-aminophenol | - | 2,0 |
| 2,4,5,6-Tetraaminopyrimidin-sulfat | - | 2,0 |
| 1,3-Bis-(2,4-diaminophenoxy)-propan · 4HCl | - | - |
| Weinsäure | - | - |
| Ammoniak (25%-ig) | ad pH=9 | 7,0 |
| Wasser | ad 100 | ad 100 |

Die Direktfarbstoff-Zubereitung und die Farbverstärker-Zubereitung werden unmittelbar vor der Anwendung im Gewichtsverhältnis 1:1 gemischt.

Es wurden folgende Handelsprodukte verwendet:
- Texapon®NSO:: Fettalkohol-(C₁₂₋₁₄)-polyglycolether-(2EO)-sulfat, Na-Salz (25%-ige Lösung)
- Dehyton® K:: Cocoamidopropylbetain, 30%-ige Lösung
- Eumulgin® B2:: Cetyl-/Stearylalkohol-polyglycolether (20EO)
- Latekoll® B:: Ethylacrylat-Methacrylsäure-Copolymerisat 25%-ige Dispersion

## Patentansprüche

1. Verfahren zur schonenden Färbung der Haare in zwei Stufen, **dadurch gekennzeichnet, daß** man das Haar in der ersten Stufe im Bereich des Haaransatzes mit einem Oxidationshaarfärbemittel unter Verwendung eines chemischen Oxidationsmittels und in einer zweiten Stufe in der gesamten Länge oder wenigstens in dem verbleibenden Längenbereich mit einem nichtoxidativen Haarfärbemittel, enthaltend reaktive Carbonylverbindungen in Kombination mit Amino- oder Hydroxylverbindungen, gleicher oder ähnlicher Nuance einfärbt.

2. Verfahren nach Anspruch 1, **dadurch gekennzeichnet, daß** das in der ersten Stufe zur Ansatzfärbung verwendete Oxidationshaarfärbemittel einen alkalischen pH-Wert im Bereich von 8-11 und die in der zweiten Stufe zur Längenfärbung verwendete Haarfärbezubereitung einen schwach sauren bis neutralen pH-Wert im Bereich von 4-7 aufweist.

3. Verfahren nach einem der Ansprüche 1 oder 2, **dadurch gekennzeichnet, daß** das in der zweiten Stufe zur Längenfärbung verwendete nicht oxidative Haarfärbemittel einen direktziehenden Farbstoff enthält aus einer Gruppe, bestehend aus direktziehenden Naturfarbstoffen und direktziehenden Nitroaromaten.

4. Haarfärbemittel bestehend aus den folgenden, getrennt verpackten, zu einer Verkaufseinheit zusammengefaßten Komponenten:
(A) einer Zubereitung von Oxidationsfarbstoffvorprodukten,
(B) einer Oxidationsmittel-Zubereitung,
(C¹) einer Zubereitung von reaktiven Carbonylverbindungen, und
(D¹) einer Zubereitung von Amino- oder Hydroxylverbindungen.

## Claims

1. A process for gently colouring the hair in two stages, **characterized in that**, in the first stage, the hair is coloured at its roots with an oxidation hair colorant using a chemical oxidizing agent and, in a second stage, is coloured over its entire length or at least over the remaining length with a non-oxidative hair colorant of the same or similar colour which contains reactive carbonyl compounds in combination with amino or hydroxyl compounds.

2. A process as claimed in claim 1, **characterized in that** the oxidation hair colorant used in the first stage for colouring the roots has an alkaline pH of 8 to 11 and the hair colouring preparation used in the second stage for length colouring has a weakly acidic to neutral pH of 4 to 7.

3. A process as claimed in claim 1 orf 2, **characterized in that** the non-oxidative hair colorant used in the second stage for length colouring contains a substantive dye from a group consisting of substantive natural dyes and substantive aromatic nitro compounds.

4. A hair colorant consisting of the following, separately packed components combined into a retail unit:
(A) a preparation of oxidation dye percursors,
(B) an oxidizing agent preparation,
(C¹) a preparation of reactive carbonyl compounds and
(D¹) a preparation of amino or hydroxyl compounds.

## Revendications

1. Procédé pour la teinture ménagée de cheveux en deux étapes, **caractérisé en ce qu'**on teint les cheveux, dans la première étape, au niveau de la naissance des cheveux, avec un agent de teinture par oxydation des cheveux avec utilisation d'un oxydant chimique et, dans une deuxième étape, sur toute la longueur ou au moins sur la partie restante de la longueur, avec un agent de teinture non oxydant des cheveux, contenant des composés carbonyle réactifs en association avec des composés amino ou hydroxyle de même nuance ou de nuance analogue.

2. Procédé selon la revendication 1, **caractérisé en ce que** l'agent de teinture par oxydation des cheveux utilisé dans la première étape pour la teinture de la naissance présente un pH alcalin dans la plage de 8 à 11 et la préparation pour la teinture de cheveux utilisée dans la deuxième étape pour la teinture de la longueur présente un pH faiblement acide à neutre dans la plage de 4 à 7.

3. Procédé selon l'une quelconque des revendications 1 ou 2, **caractérisé en ce que** l'agent de teinture pour cheveux non oxydant, utilisé dans la deuxième étape pour la teinture de la longueur contient un colorant montant directement sur la fibre provenant d'un groupe constitué par les colorants naturels montant directement sur la fibre et par les aromatiques nitro montant directement sur la fibre.

4. Agent de teinture pour cheveux constitué par les composants suivants, emballés séparément, rassemblés en une unité de vente :
(A) une préparation de précurseurs des colorants par oxydation
(B) une préparation d'oxydant
(C¹) une préparation de composés carbonyle réactifs et
(D¹) une préparation de composés amino ou hydroxyle.
